# EUROPEAN PATENT SPECIFICATION

(11) **EP 2 361 632 B1**
(45) Date of publication and mention of the grant of the patent: **19.03.2014**
(21) Application number: 10001725.0
(22) Date of filing: 19.02.2010
(51) Int. Cl.: A61K 35/00, A61K 39/02

(54) **Specific environmental bacteria for the protection from and/or the treatment of allergic, chronic inflammatory and/or autoimmune diseases**
Spezifische Umgebungsbakterien zum Schutz vor und/oder zur Behandlung von allergischen, chronischen Entzündungs- und/oder Autoimmunerkrankungen
Bactérie environnementale spécifique pour la protection contre le traitement des maladies allergiques, inflammatoires chroniques et/ou auto-immunes

(43) Date of publication of application: 31.08.2011
(73) Proprietor: Protectimmun GmbH, 45886 Gelsenkirchen (DE); Ludwig-Maximilians-Universität München, 80539 München (DE); Technische Universität München, 80333 München (DE)
(72) Inventor: Bauer, Johann, 85737 Ismaning (DE); von Mutius, Erika, 82319 Leutstetten/ Starnberg (DE); Ege, Markus, 80333 München (DE); Mayer, Melanie, 85354 Freising (DE)
(74) Representative: Godemeyer Blum Lenze - werkpatent

(56) References cited:
- EP-A1- 1 002 539
- EP-A2- 0 024 941
- WO-A1-01/49319
- US-B1- 6 187 800
- KORTHALS ET AL: "Application of PCR-SSCP for molecular epidemiological studies on the exposure of farm children to bacteria in environmental dust" JOURNAL OF MICROBIOLOGICAL METHODS, ELSEVIER, AMSTERDAM, NL LNKD- DOI:10.1016/J.MIMET.2008.01.010, vol. 73, no. 1, 5 February 2008 (2008-02-05), pages 49-56, XP022537393 ISSN: 0167-7012
- CHEN SHIXI ET AL: "A Highly Pathogenic Strain of Staphylococcus sciuri Caused Fatal Exudative Epidermitis in Piglets" PLOS ONE, vol. 2, no. 1, January 2007 (2007-01), XP002590266 ISSN: 1932-6203
- MICHEL OLIVIER: "Role of house-dust endotoxin exposure in aetiology of allergy and asthma" MEDIATORS OF INFLAMMATION, vol. 10, no. 6, December 2001 (2001-12), pages 301-304, XP002590267 ISSN: 0962-9351
- OTTAVIANI S ET AL: "Polyarticular septic arthritis due to Moraxella canis revealing multiple myeloma" JOINT BONE SPINE, ELSEVIER, PARIS, FR LNKD- DOI:10.1016/J.JBSPIN.2008.09.015, vol. 76, no. 3, 1 May 2009 (2009-05-01), pages 319-320, XP026089269 ISSN: 1297-319X [retrieved on 2009-03-14]
- SCHWAIGER K ET AL: "Notes on the almost unknown genus Jeotgalicoccus" LETTERS IN APPLIED MICROBIOLOGY, vol. 50, no. 4, April 2010 (2010-04), pages 441-444, XP002590268 ISSN: 0266-8254

## Description

The present invention relates to a composition for the protection from and/or the treatment of allergic, chronic inflammatory and/or autoimmune diseases comprising at least one specific environmental bacterium. The present disclosure further relates to a method for identifying bacteria effective in the protection from and/or the treatment of allergic diseases, chronic inflammatory and/or autoimmune diseases as well as to the use of such a bacterium/such bacteria for preparing a medicament, a pharmaceutical composition, a food additive or food ingredient, or a composition for indoor air distribution.

Overreactions of the body, particularly of the immune system, against heterogeneous (foreign) non-injurious compounds are known as allergic reactions or allergic diseases. Several types of allergic immune responses can be distinguished. Allergic reactions of type I involve e.g. hay fever, bronchial asthma, atopic dermatitis, urticaria, and food allergy. Allergic reactions of type I are mediated by IgE antibodies that are initially induced by atopic sensitization, i.e. atopic sensitization is the first incident for the development of type I allergies later in life. Even though the reasons for the occurrence and development of hypersensitivities are not fully understood up to now, it is clear that the development of the immune system is influenced by both, host and environmental factors. According to the hygiene hypothesis a low frequency of infections and a low microbial exposure during the first years of life may later lead to increased allergic reactions. For an adequate prevention, however, it is important not only to identify risk factors, but also possible protective factors.

Childhood exposure to farming has consistently been inversely associated with atopy and asthma in various regions of the world, and WO 01/49319 and EP 1 637 147 describe a composition or an extract of stable dust for prevention or treatment of allergic diseases.

EP 1 964 570 describes the use of *Acinetobacter* and *Lactococcus* for the protection against allergic diseases.

The majority of the existing studies on the effect of microbial exposure on the prevalence of allergies and related diseases were based on the analysis of microbial counts to quantify microbial exposure of humans using conventional culturing and measurements of microbial compounds such as endotoxin. These methods, however, do not reflect the total microbial diversity, as traditional microbiological techniques based on cultivation are limited in their potential to detect environmental microorganisms. This is due to the fact, that more than 90% of all microorganisms in nature cannot be isolated with common growth substrates and culture conditions. A study of Korthals et al., published in J. Microbio/. Meth. 2008, 73, 49-56, thus used a modified polymerase chain reaction single-strand conformation polymorphism protocol (PCR-SSCP) to identify bacterial communities in environmental dust.

However, in order to obtain a comprehensive protection from and/or treatment of allergic, chronic inflammatory and/or autoimmune diseases, a systematic approach not only for screening environmental bacterial exposure, but also for the correlation of the exposure with the prevalence for the respective diseases on a population level would be necessary.

Accordingly, the object of the present disclosure was to provide a method of specifically identifying environmental bacteria inversely associated with allergic, chronic inflammatory and/or autoimmune diseases on a population level, in particular with atopy, asthma or hay fever, and to provide a composition for the protection from and/or the treatment of said disease(s), containing said environmental bacteria inversely related to the disease(s) on a population level.

This object is met by the method and the composition of the present invention. The present invention provides a composition, comprising at least one bacterium selected from the group consisting of *Staphylococcus sciuri, Jeotgalicoccus* spp., preferably *J*. *halotolerans, J. pinnipedialis, J. psychrophilus, Salinicoccus alkaliphilus, Salinicoccus halodurans, Salinicoccus kunmingensis, Salinicoccus roseus, Macrococcus brunensis, Duganella* spp., preferably *D*. *violaceinigra, D. zoogloeoides, Moraxella boevrei, Moraxella canis, Moraxella caprae, Moraxella cuniculi, Moraxella lincolnii, Corynebacterium variabile, Corynebacterium freiburgense,* and *Zooglea* spp., preferably *Z*. *caeni, Z. ramigera, Z. resiniphila,* or membrane parts, membrane ingredients, cell wall proteins, particularly glycosylated proteins, polysaccharides, lipopolysaccharides, cytosolic proteins, or a mixture thereof for use in the protection from and/or the treatment of allergic diseases or chronic inflammatory diseases, selected from the group consisting of hay fever, food allergy, asthma, urticaria, neurodermitis, atopy, including atopic sensitisation and atopic dermatitis, contact eczema, psoriasis.

In terms of the present invention the term "fragments" comprises membrane parts and membrane ingredients, cell wall proteins, particularly glycosylated proteins, polysaccharides, lipopolysaccharides (endotoxines), cytosolic proteins, any molecules and/or compounds and/or metabolites expressed or synthesized by the bacteria, nucleic acids and/or any other compounds/substances produced by the bacteria or forming a part of the original bacteria. As well a mixture of bacteria fragments and whole, i.e. undestroyed, bacteria may be used.

An advantage of the composition of the present invention is that all the ingredients are harmless for humans. Nevertheless, they confer prevention or protection from allergic or inflammatory diseases. The mentioned bacteria are widely distributed in the natural environment, and no adverse effect of these bacteria on the human health is confirmed. Application/administration of the composition of the present invention before any onset of an allergic or an inflammatory disease can prevent such a disease or can alleviate the violence of outbreak. This is what is meant by "protection" or "prevention".

Preferably the allergic, chronic inflammatory and/or autoimmune diseases are selected from the group consisting of hay fever, food allergy, asthma, urticaria, neurodermitis, atopy, including atopic sensitisation and atopic dermatitis, contact eczema, psoriasis, diabetes Typ 1 or 2, multiple sclerosis, rheumatoid arthritis, diseases of the thyroid gland, including Hashimoto Thyreoditis and Graves disease, and most preferably from the group consisting of atopy, including atopic sensitisation and atopic dermatitis, asthma and hay fever.

The bacterium/bacteria is/are preferably selected from the group consisting of *Staphylococcus sciuri, Jeotgalicoccus halotolerans, Salinicoccus kunmingensis, Salinicoccus roseus, Macrococcus brunensis, Duganella violaceinigra, Moraxella cuniculi,* and *Corynebacterium variabile* wherein the composition comprises at least one of said bacteria, or a combination of at least two, at least three or more of them in any possible combination.

The bacteria to be included in the composition of the present invention can be identified by the method of the present invention, which is described in detail below, i.e. by comparison of bacterial DNA isolated from mattresses of children in association with their health status as described in the examples. The study population was the large population of the German arm of an epidemiological study called PARSIFAL which included school age children living in rural and suburban areas (Alfven et al. Allergy 2006, 61, 414-412). Population characteristics are summarized in table 1.

Although there were a lot of significant differences in exposure between farm and non-farm children, only a minority of DNA bands on an analyzing electrophoresis gel varied for the health outcomes after adjustment for farming. This shows that the identified associations of the bands with the health conditions under investigation represent exposures genuinely related to the respective health conditions. In mutually adjusted analyses seven bands were inversely related to asthma, hay fever, or atopic sensitization, showing that the exposure to the underlying bacteria confers protection from these disorders. The respective bands were excised from the SSCP gels, sequenced, and identified by comparing the sequences obtained to a data base of reference sequences.

With "at least one" according to the present invention is meant that either one of the mentioned objects is included or two, three, four and so on. In particular a combination of at least two, at least three or at least four of the mentioned objects are preferred in any possible combination, particularly preferred are combinations cited below.

Microorganisms live in communities, and the analyses described in the examples revealed that each band associated with a specific disease comprised DNA from more than one bacterial species. Accordingly, the composition of the present invention preferably comprises a combination of at least two bacteria of different species and/or different genera.

Thus, the composition of the present invention preferably further comprises at least one additional bacterium selected from the group consisting of *Staphylococcus sciuri, Jeotgalicoccus* spp., *Salinicoccus* spp., *Macrococcus brunensis, Duganella* spp., *Moraxella* spp., *Corynebacterium variabile, Corynebacterium freiburgense,* and *Zooglea* spp., more preferably selected from the group consisting of *Staphylococcus sciuri, Jeotgalicoccus halotolerans, Salinicoccus kunmingensis, Salinicoccus roseus, Macrococcus brunensis, Duganella violaceinigra, Moraxella cuniculi* and *Corynebacterium variabile,* or a fragment thereof or a mixture thereof, under the proviso that the first bacterium and the second bacterium belong to different species and preferably to different genera.

Preferred combinations of bacteria include the combinations of:
- *Staphylococcus sciuri* and *Jeotgalicoccus* spp.; *Staphylococcus sciuri* and *Salinicoccus* spp.; *Staphylococcus sciuri* and *Macrococcus brunensis; Staphylococcus sciuri* and *Duganella* spp.; *Staphylococcus sciuri* and *Moraxella* spp.; *Staphylococcus sciuri* and *Corynebacterium variabile; Staphylococcus sciuri* and *Corynebacterium freiburgense; Staphylococcus sciuri* and *Zooglea spp.;*
- *Jeotgalicoccus spp.* and *Salinicoccus spp.; Jeotgalicoccus spp.* and *Macrococcus brunensis; Jeotgalicoccus spp.* and *Duganella spp.; Jeotgalicoccus spp.* and *Moraxella spp.; Jeotgalicoccus spp.* and *Corynebacterium variabile; Jeotgalicoccus spp.* and *Corynebacterium freiburgense; Jeotgalicoccus spp.* and *Zooglea spp.;*
- *Salinicoccus spp.* and *Macrococcus brunensis; Salinicoccus spp.* and *Duganella spp.; Salinicoccus spp.* and *Moraxella boevri; Salinicoccus spp. and Moraxella canis; Salinicoccus spp. and Moraxella caprae; Salinicoccus spp. and Moraxella cuniculi; Salinicoccus spp. and Moraxella lincolnii; Salinicoccus spp. and Corynebacterium variabile; Salinicoccus spp.* and *Corynebacterium freiburgense; Salinicoccus spp.* and *Zooglea spp.;*
- *Macrococcus brunensis* and *Duganella spp.; Macrococcus brunensis* and *Moraxella spp.; Macrococcus brunensis* and *Corynebacterium variabile; Macrococcus brunensis* and *Corynebacterium freiburgense; Macrococcus brunensis* and *Zooglea spp.;*
- *Duganella spp.* and *Moraxella spp.; Duganella spp.* and *Corynebacterium variabile; Duganella spp.* and *Corynebacterium freiburgense; Duganella spp.* and *Zooglea spp.;*
- *Moraxella spp.* and *Corynebacterium variabile; Moraxella spp.* and *Corynebacterium freiburgense; Moraxella spp.* and *Zooglea spp.;*
- *Corynebacterium variabile* and *Corynebacterium freiburgense; Corynebacterium variabile* and *Zooglea spp.;*
- *Corynebacterium freiburgense* and *Zooglea spp.;*
- *as well* as *any of the above combinations with at least one further bacteria of the group comprising Staphylococcus sciuri, Jeotgalicoccus* spp., *Salinicoccus* spp., *Macrococcus brunensis, Duganella* spp., *Moraxella* spp., *Corynebacterium variabile, Corynebacterium freiburgense, Zooglea* spp., more preferably selected from the group consisting of *Staphylococcus sciuri, Jeotgalicoccus halotolerans, Salinicoccus kunmingensis, Salinicoccus roseus, Macrococcus brunensis, Duganella violaceinnigra, Moraxella cuniculi* and *Corynebacterium variabile,* or a fragment thereof or a mixture thereof, again under the proviso that the third bacterium belong to different species and preferably to different genera compared to the first and the second bacterium.

The composition of the present invention can further comprise at least one additional bacterium selected from the group consisting *of Lactobacillus* spp., preferably *Lactobacillus lactis, Lactobacillus curvatus, Lactobacillus sakei,* and *Lactobacillus iners, Delftia spp., preferably D. tsuruhatensis, Brevibacterium spp., preferably B. iodinum, B. linens, Rhizobium spp., preferably R. gallicum, Psychromonas spp., Alteromonas spp., Lactococcus lactis,* and *Acinetobacter* spp., preferably *Acinetobacter Iwoffii.*

Preferably, combinations of at least two of the bacteria identified in the bands associated with the same disease according to table 2 may be used in a protection from and/or the treatment of the disease associated with said bands.Thus, in a preferred embodiment at least two, more preferably at least three, even more preferably at least four, and most preferably all of the bacteria selected from the group consisting of *Zooglea spp., Duganella spp., L. curvatus, Moraxella spp., Staphylococcus sciuri, Jeotgalicoccus spp., Salinococcus spp.,* and *Macrococcus spp. brunensis* may be used in a composition for the protection from and/or the treatment of asthma. Preferred species of the genera are as mentioned above.

For the prevention from and/or the treatment of hay fever a combination of *Corynebacterium freiburgense* and *Corynebacterium variabile* may be used in a preferred embodiment.

For the prevention from and/or the treatment of atopic sensitization a combination of *Lactobacillus iners* and *Acinetobacter Iwoffii* may be used in a preferred embodiment.

Preferably (all of) the bacterium/bacteria comprised in the composition of the present invention is/are (a) naturally occurring non-genetically engineered and non-transgenic bacterium/bacteria, preferably (a) naturally occurring non-genetically engineered and non-transgenic bacterium/bacteria used in an isolated form.

In terms of the present invention the term "naturally occurring" bacteria refers to a bacterium/bacteria which is/are not genetically engineered and may in principle be isolated from any natural source. However, the specific bacteria comprised in the composition of the present invention may as well be grown and cultivated under defined conditions, e.g. as cell cultures, or may be obtained from commercial sources, as long as the can be found in natural sources as well.

Preferably the bacteria are used in isolated form. This means that either at least one bacteria species or a mixture of bacteria species in isolated and optionally purified form, for example in the form of an extract from a natural source, are added to the composition. Further, not only whole, i.e. undestroyed/unfragmented bacteria, but also fragments of the bacteria, including compounds/substances produced by the bacteria may be used.

Non-fragmented, i.e. whole bacteria may be used as living organisms or after any inactivation and/or denaturation step. Since the mentioned bacteria themselves are harmless forhumans, their use in vital form corresponds to natural occurrence and is preferred in terms of the present invention. To avoid contamination of the composition with other microorganisms less harmless, a step of sterilizing the composition may be applied, using for example an autoclave, or by cooking or heating the organisms, use of bactericides, bacteriastatica, fungicides, fungistatica, viricides and/or viristatica, UV rays or use of organic solutions like e.g. alcohols, particularly ethanol, propanol, isopropanol etc., lyophilisation or sterilisation by coldness.

In addition, the composition of the present invention preferably may comprise at least one additional compound selected from the groups consisting of arabinogalactans, arabinogalactan proteins, and humic acid.

The composition of the present invention preferably further may comprise at least one additional ingredient, preferably selected from the group consisting of preservatives, stabilizers, pharmaceutically acceptable carriers, emulsifiers, solvents, diluents, salts, preferably sodium chloride, flavors, fragrances and dyes.

The composition of the present invention further may comprise a carrier, either in the form of a liquid carrier, preferably in the form of a solvent, more preferably selected from the group consisting of water, isotonic saline, ethanol, propanol, and isopropanol, and mixtures thereof or in the form of a solid carrier, preferably selected from the group consisting of ground silicate, starch, cellulose, modified starch, modified cellulose, and mixtures thereof, without being limited to these.

In addition, either each of the components or the ready-prepared composition may be lyophilized to provide a storage stable product for direct application or for uptake with water or any other suitable solvent before use.

Diseases which can be prevented or treated by the composition of the present invention are particularly allergic and chronic inflammatory diseases, like IgE-depending Type I allergic diseases or Type IV allergic diseases and chronic inflammatory diseases or autoimmune diseases. Examples include hay fever, food allergy, asthma, urticaria, neurodermitis, atopic dermatitis, contact eczema, psoriasis, diabetes type 1 or 2, multiple sclerosis, rheumatoid arthritis, diseases of the thyroid gland like Hashimoto Thyreoditis and Graves disease.

Thus, in a preferred embodiment of the present invention the composition is provided as a pharmaceutical composition, preferably in the form of an aerosol, a solution, preferably an aqueous or aqueous-alcoholic solution, a suspension, a lyophilisate, a powder, a tablet, a dragee, or a suppository without being limited to the mentioned. Most of these embodiments are particularly suitable for nasal, oral, or inhalative application, and the most suitable dosage form for a particular administration mode is well known to a person skilled in the art. Most preferably the composition of the present invention is provided in the form of an aerosol having a particle size of up to 100 µm, more preferably of up to 10 µm, and most preferably of up to 5 µm.

In terms of the present invention, an aerosol comprises a plurality of small solid or liquid particles/droplets, which can be generated by any device known from the state of the art for this purpose, including an inhalator, a fogging device, or a respiratory device, without being limited to them. To support the formation of finely divided particles/droplets, a physicologically acceptable propellant, such as for example propane, butane, carbon dioxide, and the like, or a mixture of such propellants may be used. The particles/droplets of the aerosol may consist of the composition or may comprises the composition in combination with a suitable carrier. The aerosol can have a particle size e.g. of up to 100 µm for nasal application. For inhalative use the aerosol preferably has a particle size of up to 10 µm, more preferably of up to 5 µm.

In case that the bacteria are used in form of living organisms, undestroyed but denaturated organisms or insoluble fragments thereof, the composition can be in form of a suspension. For suspending the bacteria any of the solvents or solutions described above may be used.

Further, the composition can be a lyophilisate or a powder. It also may be mixed with a pharmaceutically applicable powder. Such a lyophilisate or powder can be combined with any further pharmaceutically acceptable ingredients, e.g. for application with a powder inhalator. Furthermore the powder or lyophilisate can be used to prepare a tablet or suppository for oral, anal or vaginal application.

The composition may be applied by any commonly known route, preferably by an inhalator, evaporator, fogging device, atomizer or respiratory device. It can be applied periodically over a longer time period to obtain a continued stimulation in the organism. E.g. the composition or the active ingredients can be applied up to 10 years once up to 21 times per week, preferably 7 times to 14 times per week. Preferably, the pharmaceutical composition of the present invention is applied by injection of a spray in the nose or by inhalation. In the later case time of inhalation may range from 1 to 120 minutes, preferably from 5 to 60 minutes.

The pharmaceutical composition of the present invention may be applied by any conventional application route. Application can be via oral, nasal, conjunctival, inhalative, subcutaneous, intraarticular, intraperitoneal, rectal, or vaginal route. An oral, nasal, or inhalative application route is preferred.

In another preferred embodiment, the composition of the present invention represents a food additive, a food ingredient, or a composition suitable to be distributed in the indoor air.

The composition is suitable and can serve for modulating the infant and adult immune system. Therefore the composition of the present invention can be applied/administered to pregnant women, infants, babies, and children during the first years of life (up to 8-10 years), but also to non-pregnant adults, for protection and treatment, preferably in an repetitive manner. Preferably the composition is applied/administered to pregnant women, infants, babies, children or non-pregnant adults who according to diagnostic features have an increased risk to develop any allergic or chronic inflammatory disease. The composition can also be applied to pregnant women, infants, babies, children, or non-pregnant adults who already show first signs of an allergic or chronic inflammatory disease. An increased risk as well can be determined by genetic analysis.

The composition can be applied as early as immediately after birth of a child or during the first life period. Prevention for infants may already be obtained by application of the composition of the present invention to the pregnant mother. For prevention the composition of the present invention preferably is applied to pregnant women or infants and children within the first five years of life, more preferably within the first two years of life. The composition is suitable for the application/administration to infants, babies, and/or pregnant women, preferably to such infants, babies, and/or pregnant women, which due to a positive family anamnesis have an increased risk for developing any allergic or chronic inflammatory disease or which show first signs of such disease.

For prevention the composition of the present invention is applied/administered in a amount of 1 ng to 100 g/day or 10² to 10¹¹ cfu/day, respectively. Preferably the components are applied in a amount of 1 µg to 10 g/day or 10⁴ to 10¹⁰ cfu/day (colony-forming units), respectively, referring to the combined amount of bacteria or their fragments in the composition. If the composition comprises a combination of different species, at least 10² bacteria of each species may preferably be comprised in a daily dose.

The present invention further provides a method for identifying bacteria effective in the protection from and/or the treatment of allergic, chronic inflammatory and/or autoimmune diseases, preferably selected from the group consisting of hay fever, food allergy, asthma, urticaria, neurodermitis, atopy, including atopic sensitisation and atopic dermatitis, contact eczema, psoriasis, diabetes Typ 1 or 2, multiple sclerosis, rheumatoid arthritis, diseases of the thyroid gland, including Hashimoto Thyreoditis and Graves disease, most preferably selected from the group consisting of atopy, including atopic sensitisation and atopic dermatitis, asthma and hay fever, or fragments thereof or a mixture thereof, comprising the steps of :
i) collecting dust samples from the environment of both, a) a group of individuals suffering from said diseases, and b) a group of individuals not suffering from said diseases,
ii) extracting the bacterial DNA from said samples,
iii) amplifying at least a fragment of the bacterial 16S rRNA sequences present in the sample,
iv) assessing the diversity and specificity of the bacterial community to obtain a genetic fingerprint of the bacterial environment of said individuals, preferably by single strand configuration polymorphism (SSCP),
v) determining the specific differences in the bacterial environment between group a) and group b), preferably by statistically analyzing the differences in the genetic fingerprints of group a) and b), and
vi) isolating, cloning and sequencing the DNA sequences differing in group a) and b).

The samples may be collected by any method known in the state of the art for this purpose, for example by using an adequate vacuum cleaner. Preferably, the dust samples are collected from the individuals' households, more preferably from the individual's mattresses. Mattress dust samples do far better represent the individual's exposure to a microbial environment than for example stable dust samples, since the latter do not reflect the frequency of stable visits and the intensity of microbial exposure. In addition, mattresses are able to "store" microorganisms, thus allowing to study the long time microbial exposure of an individual. In parallel, data concerning the individual's health status as well as his family anamnesis and further potential risks factors, such as maternal smoking, are collected and associated with the dust samples.

The (bacterial) DNA is extracted from the dust samples, using any method known in the state of the art for this purpose.

According to the present invention it is preferred to then amplify a suitable fragment of the bacterial 16S rRNA DNA by PCR. Preferably a fragment comprising about 350 to 450 bp of the variable regions 4 and 5 of the bacterial 16S rRNA gene is amplified, preferably by using primers which bind to the highly conserved regions of the gene. The reverse primers preferably are phosphorylated at the 5' terminus, so that the phosphorylated strand can be digested after the reaction selectively, for example by a Lamdba exonuclease.

The non-phosphorylated single strands remaining after the exonuclease digestion step show sequence-specific conformation polymorphisms under non-denaturating conditions, and may accordingly be separated by gel electrophoresis. The DNA bands obtained in the gel may be visualized by staining, for example using silver staining. This SSCP technique described above allows to assess the diversity and specificity of the bacterial community and to obtain a genetic fingerprint of the bacterial environment.

The stained gels may then be scanned and normalized using any suitable software known in the state of the art for this purpose, for example the software GelCompare II (Applied Biomaths, Sint-Martens-Latem, Belgium). The software preferably transforms the gel into a density profile, representing the band structure.

The density profile may be analyzed by any suitable statistic software known in the art for that purpose in order to determine the differences in the genetic fingerprints of both groups, for example using the statistics software SAS (The SAS Institute, Cary, NC, USA). Bands differing in the groups and statistically identified to be associated with the respective disease may then be excised from the gel, cloned and sequenced in order to identify the microorganisms differing in the environment of both groups (healthy and non-healthy individuals), for example by comparing the obtained sequences with reference databases.

As demonstrated by the examples, using the SSCP technique as a screening method on a molecular level in a large population study reveals several bands inversely related in a quantitative or qualitative manner with asthma, atopic sensitization, and hay fever. The inverse relationship strongly indicates an asthma or atopy protective potential of the bacteria represented by those bands.

As expected, farm and non-farm children differed in their bacterial exposure as determined in mattress dust samples. More than half of all bands were more common in farm children, thereby reflecting the broader spectrum of bacterial exposure in this group. Intriguingly only a minority of bands varied for the health outcomes in the farming adjusted models. This shows that the identified associations of the bands with the health conditions under investigation were not confounded by farming. In contrast, they represent exposures genuinely related to the respective health conditions.

Sequencing of these bands mainly revealed environmental bacteria (see table 2). For *Acinetobacter Iwoffii* (band 539), the biological plausibility of the association with atopic sensitization is obvious as its atopy protective potential has already been demonstrated *in vitro* and *in vivo* (Debarry et al. J. Allergy Clin. Immunol. 2007, 119, 1514-1521). Incubation with A. *Iwoffii* resulted in Th1 priming of human dendritic cells as determined by IL12 production and Notch ligand expression. In a mouse model of allergic asthma, intranasal application of A. *Iwoffii* suppressed eosinophil airway inflammation as determined in bronchoalveolar lavage and lung histology. Similar effects were found in mice exposed to *Lactococcus lactis* strains (Debarry et al. J. Allergy Clin. Immunol. 2007, 119, 1514-1521) or *Lactobacillus rhamnosus* GG (Blumer et al. Clin. Exp. Allergy 2007, 37, 348-357).

The bacteria identified by this methods or a fragment thereof are then preferably included in a composition for protection from and/or treatment of allergic, chronic inflammatory and/or autoimmune diseases, preferably for the protection from and/or the treatment of hay fever, food allergy, asthma, urticaria, neurodermitis, atopy, including atopic sensitisation and atopic dermatitis, contact eczema, psoriasis, diabetes Typ 1 or 2, multiple sclerosis, rheumatoid arthritis, diseases of the thyroid gland, including Hashimoto Thyreoditis and Graves disease. Said composition preferably is a pharmaceutical composition, preferably in the form of an aerosol, a solution, a suspension, a lyophilisate, a powder, a tablet, a dragee, or a suppository, for oral, nasal, conjunctival, inhalative, subcutaneous, intraarticular, intraperitoneal, rectal, or vaginal administration, preferably for oral, nasal or inhalative application or the composition is a food additive, a food ingredient or a composition suitable to be distributed in the indoor air.

The invention further comprises the use of at least one bacterium selected from the group consisting of *Staphylococcus sciuri, Jeotgalicoccus* spp., preferably *J*. *halotolerans, J. pinnipedialis, J. psychrophilus, Salinicoccus* spp., preferably *S*. *alkaliphilus, S. halodurans, S. kunmingensis, S*. *roseus, Macrococcus brunensis, Duganella* spp., preferably *D. violaceinigra, D. zoogloeoides, Moraxella* spp., preferably *M*. *boevrei, M. bovis, M. canis, M. caprae, M. cuniculi, M. lincolnii, Corynebacterium variabile, Corynebacterium freiburgense,* and *Zooglea* spp., preferably *Z*. *caeni, Z. ramigera, Z. resiniphila* for preparing a medicament, a pharmaceutical composition, a food additive or ingredient or a composition for indoor air distribution.

### Figures

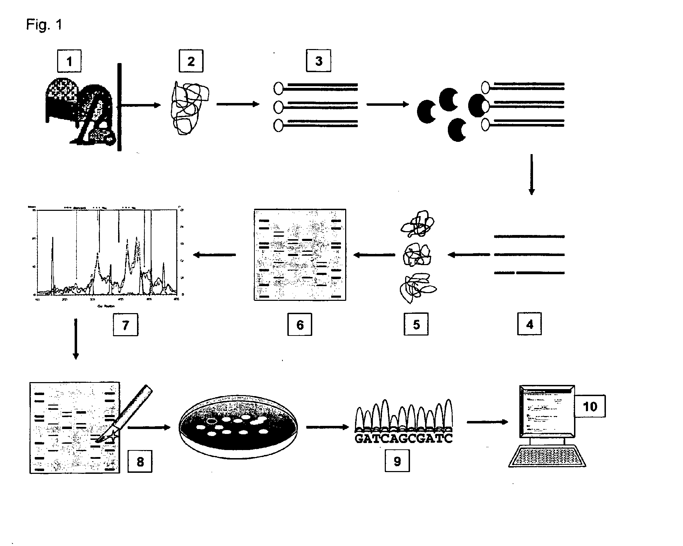

Figure 1 illustrates the steps according to a preferred embodiment of the method for identifying bacteria effective in the protection from and/or the treatment of allergic, chronic inflammatory and/or autoimmune diseases according to present invention: After collecting the respective dust samples from the environment of both, a group of individuals suffering from one or more allergic, chronic inflammatory and/or autoimmune diseases, for example from the individuals' mattress (item 1), the DNA is extracted from these samples (item 2). The bacterial 16S rRNA genes are amplified to obtain double stranded DNA (item 3). Exonuclease digestion is carried out to obtain single stranded DNA (item 4), which then is denaturated (item 5) and subjected to SSCP gel electrophoresis (item 6). The obtained gel is scanned, and the stain intensity of the bands is transformed into density units (item 7). By comparing the band densities, bands associated with the diseases mentioned above are identified by statistic analysis. The bands of interest are then excised from the gel (item 8). The DNA comprised in these bands is cloned, and then sequenced (item 9). The sequences are identified by comparison with databases to identify the specific bacteria genera and/or species (item 10).

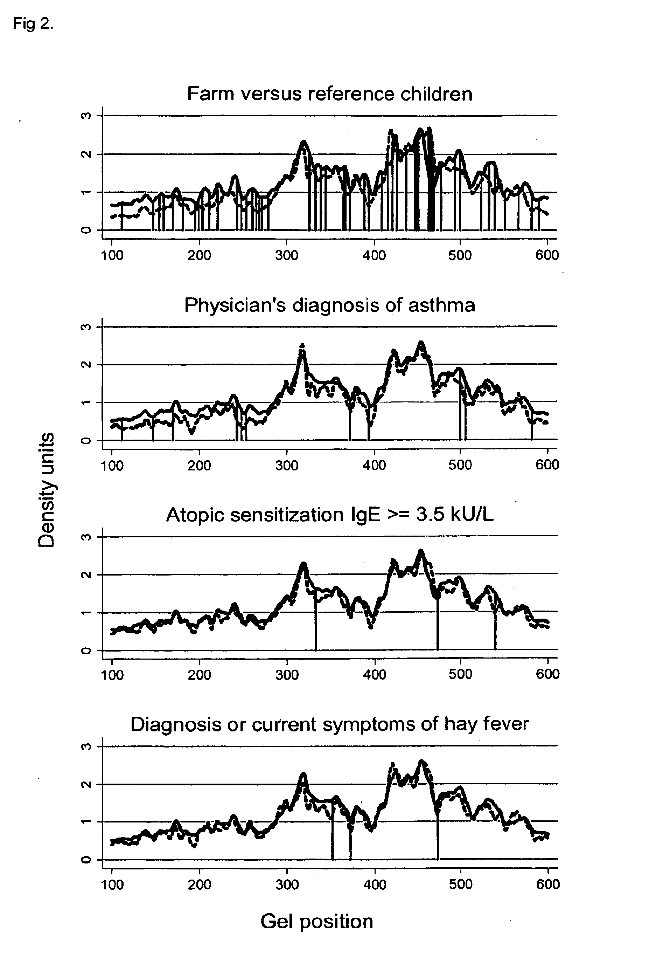

Figure 2 shows the quantitative analysis of SSCP gel densities and bands associated with childhood asthma, hay fever, and atopy (see examples). The density of silver stained gels is plotted against the position in the running direction of the gel. The uppermost panel shows mean values of log-transformed density units for farm (solid line) versus reference children (dotted line). The lower three panels give the mean values for children with (dotted line) or without (solid line) the indicated health condition. Bands with p-values <0.05 in adjusted robust logistic regression models are marked with vertical lines.

### Examples

### Example 1: Population

The PARSIFAL study was a cross-sectional survey on children of farmers, children attending Rudolf Steiner schools, and their respective reference groups. The study was carried out as described previously (Alfven et al. Allergy 2006, 61, 414-412). In the German arm of the study, 9240 children from rural or suburban areas were invited to participate. Of these, 6963 children (75%) returned their questionnaires, and 6843 (74%) had complete data for sex and age. For blood and dust analyses random samples of all children who consented to the respective study modules were selected within the four strata of farm children, children attending Rudolf Steiner schools, and their respective reference groups. Altogether 1119 children were included in blood analyses and 801 thereof additionally in dust analyses. For the dust analyses all farm children were invited. The amount of dust was sufficient for SSCP analysis in 489 children (a number of samples had been withdrawn for a study on non-atopic wheezing previously). The characteristics of the study population are summarized in table 1. The study was approved by the ethical committee of the physicians' board of Bavaria and informed consent was obtained from the children's parents or guardians for questionnaires, blood and dust samples. The questionnaire included questions on socio-demographic background, family history of asthma and atopy, exposure to farm environment, and on child's health. Questions on health outcomes were derived from the internationally validated ISAAC II questionnaire (Asher et al. Eur. Respir. J. 1995, 8, 483-491). Children with reported physician-diagnosed asthma once or obstructive bronchitis more than once in their lifetime were defined as having asthma ever. Wheezing during the past 12 months was considered as current wheeze. A child who lived on a farm and whose family ran the farm was coded as being a farm child, whereas all other children were considered reference children.

**Table 1: Characteristics of the study population**

| | **German PARSIFAL population (N=6843)** | | |
|---|---|---|---|
| **Characteristic** | **included in SSCP analysis (n = 489)** | **not included in SSCP analysis (n = 6354)** | **p-value** |
| being a farm child | 52% | 11% | <0.001 |
| age in years (mean ± standard error of mean) | 8.7 ± 0.1 | 8.9 ± 0.0 | 0.004 |
| female gender | 46% | 49% | 0.276 |
| at least one older sibling | 58% | 58% | 0.772 |
| low parental education | 30% | 29% | 0.725 |
| medium parental education | 43% | 41% | 0.407 |
| high parental education | 28% | 30% | 0.212 |
| maternal smoking during pregnancy | 4% | 9% | <0.001 |
| physician's diagnosis of asthma | 8% | 9% | 0.534 |
| wheezing during last 12 months | 3% | 8% | <0.001 |
| atopic sensitization IgE ≥ 3.5 kU/L | 17% | 18% | 0.719 |
| atopic sensitization IgE ≥ 0.35 kU/L | 37% | 32% | 0.108 |
| diagnosis or current symptoms of hay fever | 10% | 11% | 0.186 |
| family history of asthma | 10% | 11% | 0.337 |
| family history of atopy | 31% | 35% | 0.066 |

### Examle 2: Identification of protective bacteria

The DNA was extracted from the mattress dust samples as described in Korthals et al. Environ. Research 2008, 107, 299-304. The SSCP method was performed according to a previously published protocol (Korthals et al. J. Microbiol. Meth. 2008, 73, 49-56). In brief, a fragment of the variable regions 4 and 5 of the 16S ribosomal RNA gene was amplified using primers com1 and com2Ph as described by Schwieger and Tebbe, Appl Environ Microbiol 1998, 64, 4870-4876. PCR products were purified and digested to single stranded DNA and analyzed on SSCP gels. The bands were silver stained and dried at room temperature. The SSCP profiles were analyzed with the GelCompar II software (version 4.6; Applied Maths, Belgium). The SSCP gels were normalized by using a standard present on every gel in two or three tracks. The standard contained a mixture of DNA single-strands of the amplified fragment of the 16S ribosomal DNA of *Listeria monocytogenes, Bacillus licheniformis, Mycobacterium vaccae, Bacillus subtilis, Lactobacillus plantarum* and *Enterococcus faecalis.* Bands of interest were excised from at least three individual tracks of the SSCP gels, amplified, and sequenced as previously described (Korthals et al. J. Microbiol. Meth. 2008, 73, 49-56). The DNA sequences were analyzed for their closest relatives using the Ribosomal Database Project II for phylogenetic analysis available from the Michigan State University (http://rdp.cme.msu.edu/seqmatch/sectmatch intro.jsp). Sequences with a similarity of at least 98 % were reported.

**Table 2: Bacterial species which were inversely related to childhood asthma, hay fever, and atopy in mutually adjusted models**

| | **Band** | | **Quantitative *analysis*** | **Qualitative analysis** | |
|---|---|---|---|---|---|
| | | | | **cut off = 5 units** | **cut off = 10 units** |
| **Health outcome** | **Position** | **Bacterium** | **OR** | **OR** | **OR** |
| Physician diagnosis of asthma | 248 | *Zooglea* spp.; *Duganella* spp.; *Corynebacterium mycetoides* | 0.46 [0.24 - 0.89] | | |
| | | | p = 0.021 | | |
| | 394 | *Gardnerella vaginalis* | 0.56 [0.32 - 0.97] | 0.17 [0.04 - 0.72] | |
| | | | p = 0.037 | p = 0.017 | |
| | 427 | *Lactobacillus curvatus, sakei; Streptococcus* spp.; *Moraxella* spp | | | 0.45 [0.23 - 0.89] |
| | | | | | p = 0.022 |
| | 506 | *Staphylococcus sciuri,* spp.; *Jeotgalicoccus* spp.; | 0.58 [0.38 - 0.88] | 0.41 [0.20 - 0.83] | |
| | | *Salinococcus* spp.; *Macrococcus brunensis; Bacillus* spp. | p = 0.010 | p = 0.013 | |
| Hay fever | 352 | *Corynebacterium mucifaciens, freiburgense, variabile,* sp. | 0.61 [0.43 - 0.87] | | 0.28 [0.09 - 0.85] |
| | | *Triatoma infestans; Neisseria meningitidis, mucosa, subflava* | p = 0.007 | | |
| | | | | | p = 0.024 |
| Atopic sensitization | 333 | *Lactobacillus iners* | 0.73 [0.53 - 1.00] | | 0.37 [0.16 - 0.88] |
| | | | p = 0.050 | | |
| | | | | | p = 0.025 |
| | 539 | *Acinetobacter Iwoffii,* spp. | 0.65 [0.47 - 0.89] | 0.53 [0.29 - 0.98] | |
| | | | p = 0.009 | p = 0.042 | |

### Example 3: Statistical analysis

The SSCP density profiles were imported from GelCompar II as text files into SAS 9.2 (The SAS Institute, Cary, NC, USA) for statistical analysis. To assess a potential selection bias of the SSCP sample within the PARSIFAL population, children included and not included in the SSCP analysis were compared by likelihood ratio tests or t-tests. Furthermore, all children included in the SSCP analysis were compared with respect to being a farm child or not. Variables associated with inclusion in the SSCP sample or with being a farm child were then tested for their potential to confound the associations between farming and the respective health outcomes as determined by a change in the association estimate of >10%. For data reduction the SSCP gel density values at 670 gel positions were transformed to 76 band positions as follows: A band position was calculated as the maximum value of three adjacent gel positions in order to balance slight shifts caused by incomplete gel normalization. Bands with maximum density units below 5 were considered nuisance and were not included in further analyses. Because of a skewed distribution and several zero values the band density values were log-transformed after adding 1. This resulted in a good approximation to a normal distribution with some left censoring. For the quantitative analysis robust logistic regression was applied in order to minimize the effect of left censored band values. Additionally, a qualitative approach was explored by dichotomizing band densities at a low (≥ 5 density units) or high (≥ 10 density units) cut off. In a first step each band was tested for the respective health outcomes with adjustment for farming and maternal smoking. In a second step all bands with p-values < 0.05 were entered in a stepwise logistic regression with a level of stay of p < 0.05. Because some band density values were left censored a sensitivity analysis for the quantitative approach was performed. Univariate and multivariate Tobit models were explored with the band values as dependent variables and farming and the respective health outcomes as independent variables. Odds ratios (OR) from logistic regression models are given with 95% confidence intervals (CI).

In summary, Table 1 demonstrates that the samples selected for SSCP analysis comprised a high percentage of farm children in comparison to the whole PARCIFAL population. Consequently, also further characteristics related to farming such as maternal smoking or family history of atopy differed between the samples included or not included in the SSCP analysis. The samples contained almost equal ratios of asthmatics.

Within the SSCP sample, farm and non-farm children differed in the prevalence of asthma (OR = 0.56 [0.29 - 1.09]), number of older siblings, parental education, physician's diagnosis of asthma, atopic sensitization, diagnosis or current symptoms of hay fever, and family history of atopy, as previously reported (Ege et al. J. Allergy Clin. Immunol. 2006, 117, 817-823; Ege et al. J. Allergy Clin. Immunol. 2007, 119, 1140-1147). Of these variables, only farming and family history of atopy were significantly associated with a physician's diagnosis of asthma, atopic sensitization, and diagnosis or current symptoms of hay fever. The most relevant farm related exposures for asthma and atopy were regular contact to farm animals, frequent animal shed and hay loft visits, and consumption of unprocessed farm milk.

A family history of atopy confounded the associations of farming with all health outcomes. In addition high parental education confounded the association of farming with diagnosis or current symptoms of hay fever. Therefore the subsequent models were adjusted for farming, parental education, and a family history of atopy.

The presence or absence of microorganisms and probably also their quantity are related to the health outcomes. Therefore the associations of the SSCP band densities was tested quantitatively and qualitatively. For qualitative analysis two different cut offs were used, since there is no standard for dichotomization.

In the quantitative analysis farm and non-farm children differed in 51 of the 76 bands (Fig. 2, upper panel). Of these, 43 bands were more common in farm children, whereas 8 bands were more common in non-farm children. In contrast, only 11 bands were different with respect to asthma versus no asthma. All these bands were more common in children without asthma. Three bands differed with respect to atopic sensitization and diagnosis or current symptoms of hay fever, respectively. In the qualitative analysis with a cut off of 5 density units, 40 bands differed for farming, three for asthma and atopic sensitization and none for hay fever. At the higher cut off, 36 bands differed for farming, three for asthma, eight for atopic sensitization and two for hay fever. Only in quantitative models bands with positive associations with the health conditions were detected: two for asthma, two for sensitization and one for hay fever (data not shown).

## Claims

1. A composition, comprising at least one bacterium selected from the group consisting of *Staphylococcus sciuri, Jeotgalicoccus* spp., preferably *J*. *halotolerans, J. pinnipedialis, J. psychrophilus, Salinicoccus alkaliphilus, Salinicoccus halodurans, Salinicoccus kunmingensis, Salinicoccus roseus, Macrococcus brunensis, Duganella* spp., preferably *D*. *violaceinigra, D. zoogloeoides, Moraxella boevrei, Moraxella canis, Moraxella caprae, Moraxella cuniculi, Moraxella lincolnii, Corynebacterium variabile, Corynebacterium freiburgense,* and *Zooglea* spp., preferably *Z*. *caeni, Z. ramigera, Z. resiniphila,* or membrane parts, membrane ingredients, cell wall proteins, particularly glycosylated proteins, polysaccharides, lipopolysaccharides, cytosolic proteins, or a mixture thereof for use in the protection from and/or the treatment of allergic diseases or chronic inflammatory diseases, selected from the group consisting of hay fever, food allergy, asthma, urticaria, neurodermitis, atopy, including atopic sensitisation and atopic dermatitis, contact eczema, psoriasis.

2. Composition for use according to claim 1, further comprising at least one additional
bacterium selected from the group consisting of *Staphylococcus sciuri, Jeotgalicoccus* spp., *Salinicoccus* spp., *Macrococcus brunensis, Duganella* spp., *Moraxella* spp., *Corynebacterium variabile, Corynebacterium freiburgense,* and *Zooglea* spp., or membrane parts, membrane ingredients, cell wall proteins, particularly glycosylated proteins, polysaccharides, lipopolysaccharides, cytosolic proteins, or a mixture thereof, under the proviso that the first bacterium and the second bacterium belong to different genera.

3. A composition for use according to claim 1 or 2, further comprising at least one additional bacterium selected from the group consisting *of Lactobacillus* spp., preferably *Lactobacillus curvatus, Lactobacillus sakei,* and *Lactobacillus iners, Delftia spp., preferably D. tsuruhatensis, Brevibacterium spp., preferably B. iodinum, B. linens, Rhizobium spp., preferably R. gallicum, Psychromonas spp., Alteromonas spp., Lactococcus lactis,* and *Acinetobacter spp.,* preferably *Acinetobacter Iwoffii.*

4. A composition for use according to any of claims 1 to 3, wherein the bacterium is a naturally occurring non-genetically engineered and non-transgenic bacterium, preferably a naturally occurring non-genetically engineered and non-transgenic bacterium used in an isolated form.

5. A composition for use according to any of claims 1 to 4, further comprising at least one additional compound selected from the groups consisting of arabinogalactans, arabinogalactan proteins, and humic acid.

6. A composition for use according to any of claims 1 to 5, further comprising at least one additional ingredient, preferably selected from the group consisting of preservatives, stabilizers, pharmaceutically acceptable carriers, emulsifiers, solvents, diluents, salts, preferably sodium chloride, flavors, fragrances, and dyes.

7. A composition for use according to claim 6, wherein the composition comprises a carrier, either in the form of a liquid carrier, preferably in the form of a solvent, more preferably selected from the group consisting of water, isotonic saline, ethanol, propanol, and isopropanol, and mixtures thereof or in the form of a solid carrier, preferably selected from the group consisting of ground silicate, starch, cellulose, modified starch, modified cellulose, and mixtures thereof.

8. A composition for use according to any of claims 1 to 7, wherein the composition is a pharmaceutical composition, preferably in the form of an aerosol, a solution, a suspension, a lyophilisate, a powder, a tablet, a dragee, or a suppository, most preferably in the form of an aerosol having a particle size of up to 100 µm, more preferably of up to 10 µm, and most preferably of up to 5 µm.

9. A composition for use according to claim 8 for oral, nasal, conjunctival, inhalative, subcutaneous, intraarticular, intraperitoneal, rectal, or vaginal administration.

10. A composition for use according to any of claims 1 to 7, wherein the composition is a food additive, a food ingredient, or a composition suitable to be distributed in the indoor air.

11. A composition for use according to any of claims 1 to 10, suitable for the application to babies, infants, or pregnant women, preferably to such babies, infants, or pregnant women which due to a positive family anamnesis have an increased risk for developing any allergic or chronic inflammatory diseases or which show first signs of such a diseases.

12. A composition comprising at least one bacterium selected from the group consisting of *Staphylococcus sciuri, Jeotgalicoccus* spp., preferably *J*. *halotolerans, J. pinnipedialis, J. psychrophilus, Salinicoccus alkaliphilus, Salinicoccus halodurans, Salinicoccus kunmingensis, Salinicoccus roseus, Macrococcus brunensis, Duganella* spp., preferably *D*. *violaceinigra, D. zoogloeoides, Moraxella boevrei, Moraxella canis, Moraxella caprae, Moraxella cuniculi, Moraxella lincolnii, Corynebacterium variabile, Corynebacterium freiburgense, Zooglea* spp., preferably *Z*. *caeni, Z. ramigera, Z. resiniphila* for use as a medicament or a pharmaceutical composition.

13. Use of at least one bacterium selected from the group consisting of *Jeotgalicoccus* spp., preferably *J*. *halotolerans, J. pinnipedialis, J. psychrophilus, Salinicoccus alkaliphilus, Salinicoccus halodurans, Salinicoccus kunmingensis, Salinicoccus roseus, Macrococcus brunensis, Duganella* spp., preferably *D. violaceinigra, D. zoogloeoides, Moraxella boevrei, Moraxella canis, Moraxella caprae, Moraxella cuniculi, Moraxella lincolnii, Corynebacterium variabile, Corynebacterium freiburgense, Zooglea* spp., preferably *Z*. *caeni, Z. ramigera, Z. resiniphila* in a food additive or ingredient or in a composition for indoor air distribution.

14. Use of *Staphylococcus sciuri* in a composition for indoor air distribution.

## Patentansprüche

1. Eine Zusammensetzung enthaltend mindestens ein Bakterium ausgewählt aus der Gruppe bestehend aus *Staphylococcus sciuri, Jeotgalicoccus* spp., vorzugsweise *J. halotolerans, J. pinnipedialis, J. psychrophilus, Salinicoccus alkaliphilus, Salinicoccus halodurans, Salinicoccus kunmingensis, Salinicoccus roseus, Macrococcus brunensis, Duganella* spp., vorzugsweise *D. violaceinigra, D. zoogloeoides, Moraxella boevrei, Moraxella canis, Moraxella caprae, Moraxella cuniculi, Moraxella fincolnii, Corynebacterium variabile, Corynebacterium freiburgense,* und *Zooglea* spp., vorzugsweise *Z*. *caeni, Z. ramigera, Z. resiniphila,* oder Membranteile, Membranbestandteile, Zellwandproteine, insbesondere glykosylierte Proteine, Polysaccharide, Lipopolysaccharide, cytosolische Proteine, oder eine Mischung davon, in der Vorbeugung von und/oder in der Behandlung von allergischen Krankheiten oder chronisch entzündlichen Krankheiten ausgewählt aus der Gruppe bestehend aus Allergische Rhinitis, Nahrungsmittelallergie, Asthma, Urtikaria, Neurodermitis, Atopie, einschließlich atopische Sensibilisierung und atopische Dermatitis, allergisches Kontaktekzem, Psoriasis.

2. Eine Zusammensetzung zur Verwendung gemäß Anspruch 1, weiterhin enthaltend mindestens ein zusätzliches Bakterium ausgewählt aus der Gruppe bestehend aus
*Staphylococcus sciuri, Jeotgalicoccus* spp., *Salinicoccus* spp., *Macrococcus brunensis, Duganella* spp., *Moraxella* spp., *Corynebacterium variabile, Corynebacterium freiburgense,* und *Zooglea* spp., oder Membranteile, Membranbestandteile, Zellwandproteine, insbesondere glykosylierte Proteine, Polysaccharide, Lipopolysaccharide, cytosolische Proteine, oder eine Mischung davon, mit der Maßgabe, dass das erste Bakterium und das zweite Bakterium unterschiedlichen Gattungen angehören.

3. Eine Zusammensetzung zur Verwendung gemäß Anspruch 1 oder 2, weiterhin enthaltend mindestens ein zusätzliches Bakterium ausgewählt aus der Gruppe bestehend aus *Lactobacillus* spp., vorzugsweise *Lactobacillus curvatus, Lactobacillus sakei,* und *Lactobacillus iners, Delftia spp.,* vorzugsweise *D*. *tsuruhatensis, Brevibacterium spp.,* vorzugsweise *B*. *iodinum, B. linens, Rhizobium spp.,* vorzugsweise *R. gallicum, Psychromonas spp., Alteromonas spp., Lactococcus lactis,* und *Acinetobacter spp.,* vorzugsweise *Acinetobacter Iwoffii.*

4. Eine Zusammensetzung zur Verwendung gemäß irgendeinem der Ansprüche 1 bis 3, wobei das Bakterium ein natürlich vorkommendes genetisch nicht modifiziertes und nicht-transgenes Bakterium ist, vorzugsweise ein natürlich vorkommendes genetisch nicht modifiziertes und nicht-transgenes Bakterium in isolierter Form.

5. Eine Zusammensetzung zur Verwendung gemäß irgendeinem der Ansprüche 1 bis 4, weiterhin enthaltend mindestens eine zusätzliche Substanz ausgewählt aus der Gruppe bestehend aus Arabinogalaktanen, Arabinogalaktanproteinen, und Huminsäure.

6. Eine Zusammensetzung zur Verwendung gemäß irgendeinem der Ansprüche 1 bis 5, weiterhin enthaltend mindestens einen zusätzlichen Inhaltsstoff, vorzugsweise ausgewählt aus der Gruppe bestehend aus Konservierungsmitteln, Stabilisierungsmitteln, pharmazeutisch akzeptablen Trägern, Emulgatoren, Lösungsmitteln, Verdünnungsmitteln, Salzen, vorzugsweise Natriumchlorid, Geschmacksstoffen, Duftstoffen und Farbstoffen.

7. Eine Zusammensetzung zur Verwendung gemäß Anspruch 6, wobei die Zusammensetzung einen Träger enthält, entweder in Form eines flüssigen Trägers, vorzugsweise in Form eines Lösungsmittels, weiter bevorzugt ausgewählt aus der Gruppe bestehend aus Wasser, isotonische Kochsalzlösung, Ethanol, Propanol, und Isopropanol, und Mischungen davon, oder in Form eines festen Trägers, vorzugsweise ausgewählt aus der Gruppe bestehend aus gemahlenem Silikat, Stärke, Cellulose, modifizierte Stärke, modifizierte Cellulose und Mischungen davon.

8. Eine Zusammensetzung zur Verwendung gemäß irgendeinem der Ansprüche 1 bis 7, wobei die Zusammensetzung eine pharmazeutische Zusammensetzung ist, vorzugsweise in Form eines Aerosols, einer Lösung, einer Suspension, eines Lyophilisats, eines Pulvers, einer Tablette, eines Dragees oder eines Zäpfchens, am meisten bevorzugt in Form eines Aerosols mit einer Partikelgröße bis zu 100 µm, weiter bevorzugt bis zu 10 µm, und am meisten bevorzugt bis zu 5 µm.

9. Eine Zusammensetzung zur Verwendung gemäß Anspruch 8 zur oralen, nasalen, konjunktivalen, inhalativen, subkutanen, intraartikulären, intraperitonealen, rektalen oder vaginalen Verabreichung.

10. Eine Zusammensetzung zur Verwendung gemäß irgendeinem der Ansprüche 1 bis 7, wobei die Zusammensetzung ein Nahrungsmittelzusatzstoff, ein Nahrungsmittelbestandteil, oder eine Zusammensetzung geeignet zur Verteilung in der Raumluft ist.

11. Eine Zusammensetzung zur Verwendung gemäß irgendeinem der Ansprüche 1 bis 10, die geeignet ist zur Verabreichung an Säuglinge, Kinder oder Schwangere, vorzugsweise an solche Säuglinge, Kinder oder Schwangere, die auf Grund einer positiven Familienanamnese ein deutlich höheres Risiko aufweisen, allergische oder chronisch entzündliche Erkrankungen zu entwickeln, oder die erste Manifestationen einer solchen Erkrankung aufweisen.

12. Eine Zusammensetzung enthaltend mindestens ein Bakterium ausgewählt aus der Gruppe bestehend aus *Staphylococcus scuri, Jeotgalicoccus* spp., vorzugsweise *J*. *halotolerans, J. pinnipedialis, J. psychrophilus, Salinicoccus alkaliphilus, Salinicoccus halodurans, Salinicoccus kunmingensis, Salinicoccus roseus, Macrococcus brunensis, Duganella* spp., vorzugsweise *D. violaceinigra, D. zoogloeoides, Moraxella boevrei, Moraxella canis, Moraxella caprae, Moraxella cuniculi, Moraxella lincolnii, Corynebacterium variabile, Corynebacterium freiburgense, Zooglea* spp., vorzugsweise *Z*. *caeni, Z. ramigera, Z. resiniphila* zur Verwendung als Medikament oder als pharmazeutische Zusammensetzung.

13. Verwendung von mindestens einem Bakterium ausgewählt aus der Gruppe bestehend aus *Jeotgalicoccus* spp., vorzugsweise *J. halotolerans, J. pinnipedialis, J. psychrophilus, Salinicoccus alkaliphilus, Salinicoccus halodurans, Salinicoccus kunmingensis, Salinicoccus roseus, Macrococcus brunensis, Duganella* spp., vorzugsweise *D*. *violaceinigra, D. zoogloeoides, Moraxella boevrei, Moraxella canis, Moraxella caprae, Moraxella cuniculi, Moraxella lincolnii, Corynebacterium variabile, Corynebacterium freiburgense, Zooglea* spp., vorzugsweise *Z*. *caeni, Z. ramigera, Z. resiniphila* in einem Nahrungsmittelzusatzstoff, oder als Nahrungsmittelbestandteil, oder in einer Zusammensetzung, die zur Verteilung in der Raumluft geeignet ist.

14. Verwendung von *Staphylococcus sciuri* in einer Zusammensetzung, die zur Verteilung in der Raumluft geeignet ist.

## Revendications

1. Composition comprenant au moins une bactérie sélectionnée dans le groupe constitué de *Staphylococcus sciuri, Jeotgalicoccus spp*., de préférence *J*. *halotolerans, J. pinnipediasis, J. psychrophilus, Salinicoccus alkaliphilus, Salinicoccus halodurans, Salinicoccus kunmingensis, Salinicoccus roseus, Macrococcus brunensis, Duganella spp.,* de préférence *D*. *violaceinigra, D. zoogloeoides, Moraxella boevrei, Moraxella canis, Moraxella caprae, Moraxella cuniculi, Moraxella lincolnii, Corynebacterium variabile, Corynebacterium freiburgense,* et *Zooglea spp.,* de préférence *Z. caeni, Z. ramigera, Z. resiniphila,* au des parties de la membrane, des ingrédients de la membrane, des protéines de paroi cellulaire, notamment des protéines glycosylées, des polysaccharides, des lipopolysaccharides, des protéines cytosoliques, ou d'un mélange de ceux-ci pour son utilisation dans la protection contre les maladies allergiques ou les maladies inflammatoires chroniques et/ou dans le traitement desdites maladies, lesdites maladies étant sélectionnées dans le groupe constitué du rhume des foins, des allergies alimentaires, de l'asthme, de l'urticaire, de la neurodermatose, de l'atopie, y compris la sensibilisation atopique et la dermatite atopique, de l'eczéma de contact, du psoriasis.

2. Composition pour son utilisation selon la revendication 1, comprenant en outre au moins une bactérie supplémentaire sélectionnée dans le groupe constitué de *Staphylococcus sciuri, Jeotgalicoccus spp., Salinicoccus spp., Macrococcus brunensis*, *Duganella spp*., *Moraxella spp*., *Corynebacterium variabile*, *Corynebacterium freiburgense,* et *Zooglea spp.,* ou des parties de la membrane, des ingrédients de la membrane, des protéines de paroi cellulaire, notamment des protéines glycosylées, des polysaccharides, des lipopolysaccharides, des protéines cytosoliques, ou un mélange de ceux-ci, à condition que la première bactérie et la seconde bactérie appartiennent à des genres différents.

3. Composition pour son utilisation selon la revendication 1 ou 2, comprenant au moins une bactérie supplémentaire sélectionnée dans le groupe constitué de *Lactobacillus spp*., de préférence *Lactobacillus curvatus*, *Lactobacillus sakei*, et *Lactobacillus iners*, *Delftia spp*., de préférence *D*. *tsuruhatensis, Brevibacterium spp*., de préférence *B*. *iodinum*, *B*. *linens, Rhizobium spp., preferably R*. *gallicum, Psychromonas spp., Alteromonas spp., Lactococcus lactis*, et *Acinetobacter spp.,* de préférence *Acinetobacter Iwoffii*.

4. Composition pour son utilisation selon l'une quelconque des revendications 1 à 3, dans laquelle la bactérie est une bactérie naturelle, non issue du génie génétique et non transgénique, de préférence une bactérie naturelle, non issue du génie génétique et non transgénique utilisée sous une forme isolée.

5. Composition pour son utilisation selon l'une quelconque des revendications 1 à 4, comprenant en outre au moins un composé supplémentaire sélectionné dans les groupes constitués des arabinogalactanes, des protéines arabinogalactanes, et de l'acide humique.

6. Composition pour son utilisation selon l'une quelconque des revendications 1 à 5, comprenant en outre au moins un ingrédient supplémentaire, de préférence sélectionné dans le groupe constitué des conservateurs, des agents de stabilisation, des véhicules pharmaceutiquement acceptables, des émulsifiants, des solvants, des diluants, des sels, de préférence le chlorure de sodium, des arômes, des fragrances, et des colorants.

7. Composition pour son utilisation selon la revendication 6, dans laquelle la composition comprend un véhicule, sous la forme d'un véhicule liquide, de préférence sous la forme d'un solvant, de manière davantage préférée sélectionné dans le groupe constitué de l'eau, d'une solution saline isotonique, de l'éthanol, du propanol, et de l'isopropanol, et de leurs mélanges, ou sous la forme d'un véhicule solide, de préférence sélectionné dans le groupe constitué du silicate broyé, de l'amidon, de la cellulose, de l'amidon modifié, de la cellulose modifiée, et de leurs mélanges.

8. Composition pour son utilisation selon l'une quelconque des revendications 1 à 7, dans laquelle la composition est une composition pharmaceutique, de préférence sous la forme d'un aérosol, d'une solution, d'une suspension, d'un lyophilisat, d'une poudre, d'un comprimé, d'une dragée, ou d'un suppositoire, de manière davantage préférée sous la forme d'un aérosol ayant une taille de particules d'au plus 100 µm, de préférence d'au plus 10 µm, et idéalement d'au plus 5 µm.

9. Composition pour son utilisation selon la revendication 8 pour une administration orale, nasale, conjonctivale, par inhalation, sous-cutanée, intra-articulaire, intrapéritonéale, rectale, ou vaginale.

10. Composition pour son utilisation selon l'une quelconque des revendications 1 à 7, dans laquelle la composition est un supplément alimentaire, un ingrédient alimentaire, ou une composition adaptée à être distribuée dans l'air intérieur.

11. Composition pour son utilisation selon l'une quelconque des revendications 1 à 10, adaptée à l'application à des nouveau-nés, des nourrissions, ou des femmes enceintes, de préférence aux nouveau-nés, aux nourrissons, ou aux femmes enceintes qui, en raison d'une anamnèse familiale positive, ont un risque accru de développer une maladie inflammatoire chronique ou allergique ou qui présentent les premiers signes de ces maladies.

12. Composition comprenant au moins une bactérie sélectionnée dans le groupe constitué de *Staphylococcus sciuri*, *Jeotgalicoccus spp*., de préférence *J*. *halotolerans, J. pinnipedialis, J. psychrophilus, Salinicoccus alkaliphilus, Salinicoccus halodurans, Salinicoccus kunmingensis, Salinicoccus roseus, Macrococcus brunensis, Duganella spp.,* de préférence *D*. *violaceinigra, D*. *zoogloeoides, Moraxella boevrei, Moraxella canis, Moraxella caprae, Moraxella cuniculi, Moraxella lincolnii, Corynebacterium variabile, Corynebacterium freiburgense, Zooglea spp.,* de préférence *Z*. *caeni, Z. ramagera, Z. resiniphila* pour son utilisation en tant que médicament ou composition pharmaceutique.

13. Utilisation d'au moins une bactérie sélectionnée dans le groupe constitué de *Jeotgalicoccus spp.,* de préférence *J*. *halotolerans, J. pinnipedialis, J*. *psychrophilus, Salinicoccus alkaliphilus, Salinicoccus halodurans, Salinicoccus kunmingensis, Salinicoccus roseus, Macrococcus brunensis, Duganella spp.,* de préférence *D. violaceinigra, D. zoogloeoides, Moraxella boevrei, Moraxella canis, Moraxella caprae, Moraxella cuniculi, Moraxella lincolnii, Corynebacterium variabile, Corynebacterium freiburgense, Zooglea spp.,* de préférence *Z. caeni, Z. ramigera, Z. resiniphila* dans un auditif ou ingrédients alimentaire ou dans une composition pour la distribution dans l'air intérieur.

14. Utilisation de *Staphylococcus sciuri* dans une composition pour la distribution dans l'air intérieur.
